# EUROPEAN PATENT SPECIFICATION

(11) **EP 3 638 221 B1**
(45) Date of publication and mention of the grant of the patent: **29.09.2021**
(21) Application number: 18731452.1
(22) Date of filing: 15.06.2018
(51) Int. Cl.: A61K 31/121, A61P 1/16, A61P 13/12

(54) **COMPOSITIONS AND METHODS FOR TREATMENT OF A FIBROTIC DISEASE**
ZUSAMMENSETZUNGEN UND VERFAHREN ZUR BEHANDLUNG VON FIBROTISCHEN ERKRANKUNGEN
COMPOSITIONS ET MÉTHODES POUR LE TRAITEMENT D'UNE MALADIE FIBROTIQUE

(30) Priority: 16.06.2017 GB 201709671; 24.04.2018 GB 201806661
(43) Date of publication of application: 22.04.2020
(73) Proprietor: Avexxin AS, 7052 Trondheim (NO)
(72) Inventor: JOHNASEN, Berit, 7052 Trondheim (NO); FEUERHERM, Astrid Jullumstr, 7052 Trondheim (NO); ALEVIZOPOULOS, Konstantinos, 7052 Trondheim (NO); SELVIK, Linn-Karina, 7052 Trondheim (NO)
(74) Representative: Dehns
(86) International application number: PCT/EP2018/066028
(87) International publication number: WO 2018/229284

(56) References cited:
- WO-A1-02/060535
- WO-A1-2010/139482
- WO-A1-2012/028688
- Berit Johansen et al.: "Novel inhibitor of cytosolic group IVA phospholipase A2 (cPLA2) ameliorate mesangial inflammation and kidney fibrosis development in diabetic nephropathy", LIPID MAPS Annual Meeting 2015:Lipidomics Impact on Cancer, Metabolic, and Inflammatory Diseases (12th annual meeting)May 12-13, 2015, La Jolla, CA , 12 May 2015 (2015-05-12), page Abstract 17, XP002783913, Retrieved from the Internet: URL:http://www.lipidmaps.org/meetings/2015 annual/posters.pdf [retrieved on 2018-08-15]
- ANDREA HUWILER ET AL: "The [omega]3-polyunsaturated fatty acid derivatives AVX001 and AVX002 directly inhibit cytosolic phospholipase A 2 and suppress PGE 2 formation in mesangial cells", BRITISH JOURNAL OF PHARMACOLOGY, vol. 167, no. 8, 29 December 2012 (2012-12-29), pages 1691-1701, XP55116796, ISSN: 0007-1188, DOI: 10.1111/j.1476-5381.2012.02114.x
- ZHAO LIENA ET AL: "Involvement of cytosolic phospholipase A2 alpha signalling pathway in spontaneous and transforming growth factor-beta-induced activation of rat hepatic stellate cells.", LIVER INTERNATIONAL : OFFICIAL JOURNAL OF THE INTERNATIONAL ASSOCIATION FOR THE STUDY OF THE LIVER NOV 2011, vol. 31, no. 10, November 2011 (2011-11), pages 1565-1573, XP002783914, ISSN: 1478-3231
- KHAN NAYAAB S ET AL: "Cytosolic Phospholipase A2[alpha] Is Essential for Renal Dysfunction and End-Organ Damage Associated With Angiotensin II-Induced Hypertension.", AMERICAN JOURNAL OF HYPERTENSION FEB 2016, vol. 29, no. 2, February 2016 (2016-02), pages 258-265, XP055500263, ISSN: 1941-7225

## Description

This invention relates to compositions for use in the treatment of fibrotic diseases or fibrotic disorders. In particular, the invention relates to the use of certain polyunsaturated long-chain ketones in the treatment, prevention or reduction of symptoms of fibrosis or related conditions.

### BACKGROUND OF THE INVENTION

Fibrosis is a feature of many chronic inflammatory diseases, and is an important cause of morbidity and mortality worldwide. Fibrosis is characterized by the accumulation of excess extracellular matrix components (e.g. collagen, fibronectin) that forms fibrous connective tissue in and around an inflamed or damaged tissue. Fibrosis may cause, for example, overgrowth, hardening, and/or scarring that disrupts the architecture of the underlying organ or tissue. While controlled tissue remodeling and scarring is part of the normal wound healing process, excessive and persistent scarring due to severe or repetitive injury or dysregulated wound healing can eventually lead to permanent scarring, organ dysfunction and failure, and even death.

Fibrosis and related changes can occur in vascular disorders such as peripheral vascular disease, cardiac disease, cerebral disease and in all main tissue and organ systems (e.g., lung, liver, kidney, heart, skin). Fibrotic disorders include a wide range of clinical presentations, including multisystemic disorders, such as systemic sclerosis, multifocal fibrosclerosis, and organ-specific disorders, such as pulmonary, liver, and kidney fibrosis. While the etiology and causative mechanisms of individual fibrotic disorders may vary (e.g., ischemic event, exposure to a chemical, radiation, or infectious agent) and are not completely understood, nearly all share the common feature of abnormal and excessive deposition of extracellular matrix in affected tissues.

The present inventors sought new methods for treating fibrotic diseases as there are no clearly effective therapies for treating, preventing, or reducing symptoms of fibrotic diseases. Thus, there is a need for effective methods of treating, preventing or reducing symptoms associated with these disorders. The present inventors have surprisingly found that certain polyunsaturated long-chain ketones can be used to treat, prevent or reduce symptoms of fibrotic diseases.

The polyunsaturated long-chain ketones are not themselves new. Methods for making polyunsaturated ketone compounds have been disclosed in J. Chem. Soc., Perkin Trans 1, 2000, 2271-2276. These compounds have not however, been previously suggested for use in the treatment, prevention or reduction of symptoms of fibrotic disorders.

WO 02/060535 discloses a method for treating or modulating inflammatory processes, such as idiopathic pulmonary fibrosis using a cPLA2 inhibitor.

Liver International, 2011, p.1565-1573; L. Zhao et al. describes cPLA2 inhibition in relation to liver damage and shows that cPLA2 is involved in activation of hepatic stellate cells (HSC).

### SUMMARY OF THE INVENTION

Thus, viewed from one aspect the invention provides a compound of formula (IV)

R-Y1-CH2-CO-X (IV)

wherein R is a linear C₁₀₋₂₄ unsubstituted unsaturated alkylene group said group comprising at least 4 non-conjugated double bonds;
X is CF₃; and
Y1 is selected from O, S, SO or SO₂; or a salt thereof
for use in the treatment or prevention of a fibrotic disease, wherein the fibrotic disease is pulmonary fibrotic disorder or liver fibrosis.

Viewed from another aspect the invention provides a compound of formula (IV) or a salt thereof as hereinbefore described for use for treating, preventing or reducing symptoms of a fibrotic disorder, selected from a pulmonary fibrotic disorder or liver fibrosis.

Viewed from another aspect the invention provides a compound of formula (IV) or a salt thereof as hereinbefore described for use as a medicament for reducing one or more of hydroxyproline content or collagen Type I mRNA expression in the lung or liver of an animal.

### BRIEF DESCRIPTION OF THE FIGURES

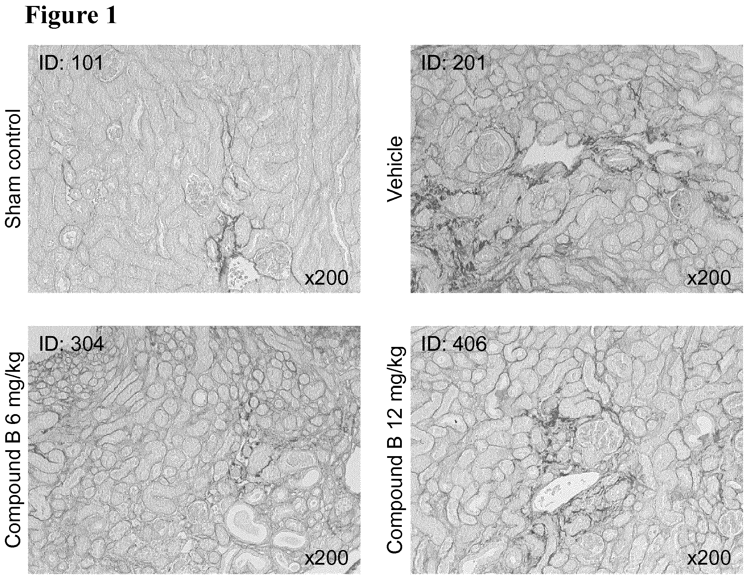

Figure 1 shows representative photomicrographs of UUO-induced kidney fibrosis revealed by Sirius red-staining of kidney sections. UUO induced significant kidney fibrosis (Vehicle group) in comparison to Sham control. Kidney fibrosis was significantly reduced in UUO animals treated with Compound B. (x200: applied magnification).

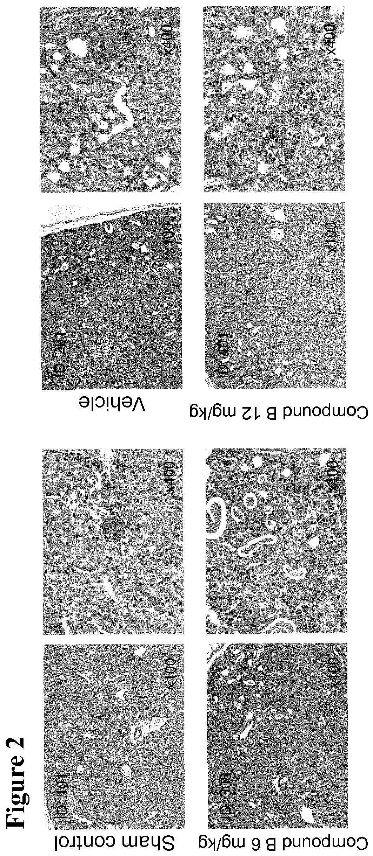

Figure 2 shows representative photomicrographs of UUO-induced kidney inflammation revealed by PAS-stained kidney sections. UUO induced significant kidney inflammation (Vehicle group) in comparison to Sham control. Kidney inflammation was reduced in UUO animals treated with Compound B, especially at the high dose group. (x100, x400: different magnifications of the same sample).

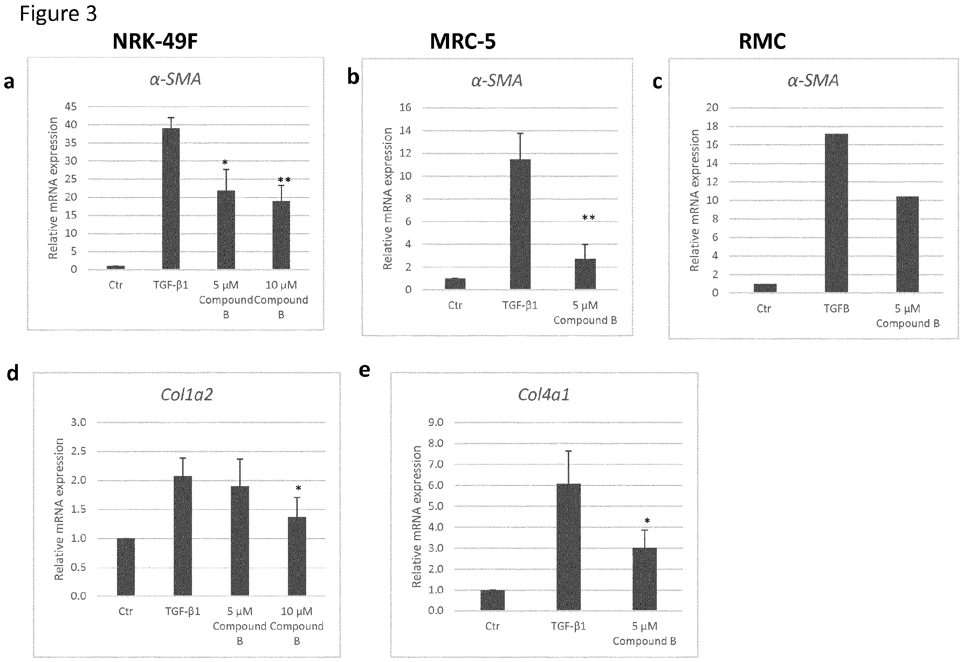

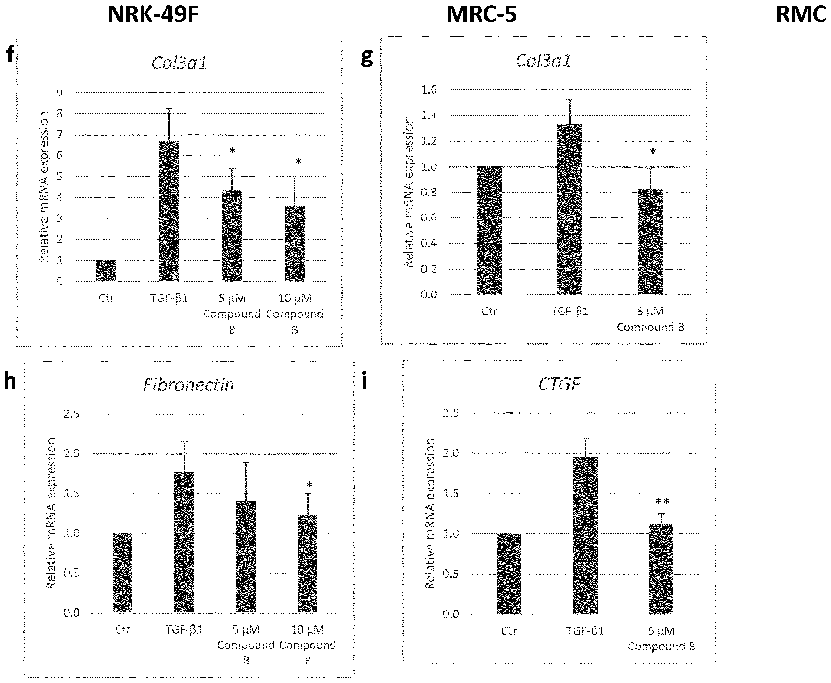

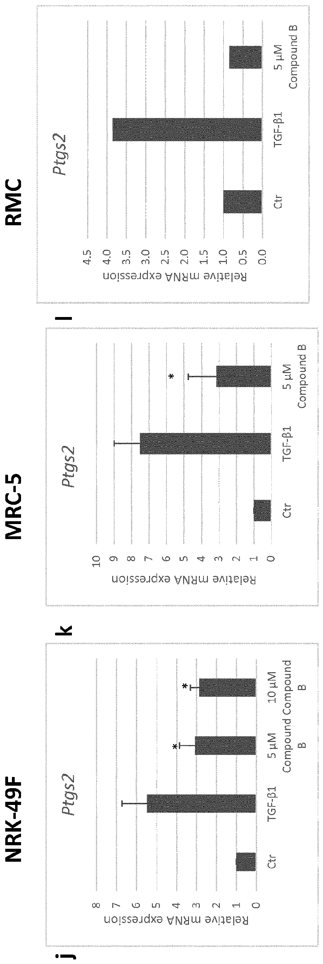

Figure 3 shows that selective inhibitors against cPLA2α reduce the mRNA expression of fibrotic markers in TGF-β1 treated cells.

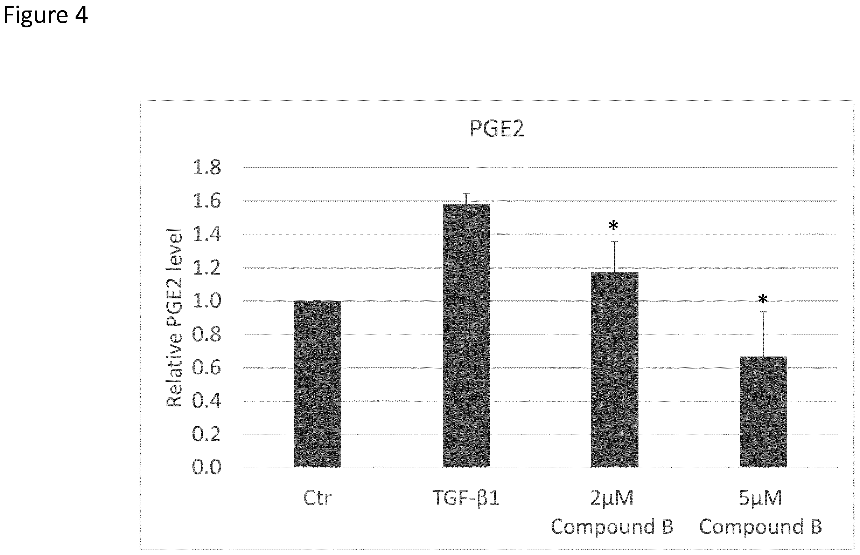

Figure 4 shows that Compound B reduces proinflammatory eicosanoid PGE2 levels in supernatants from TGF-β1 treated rat kidney fibroblasts.

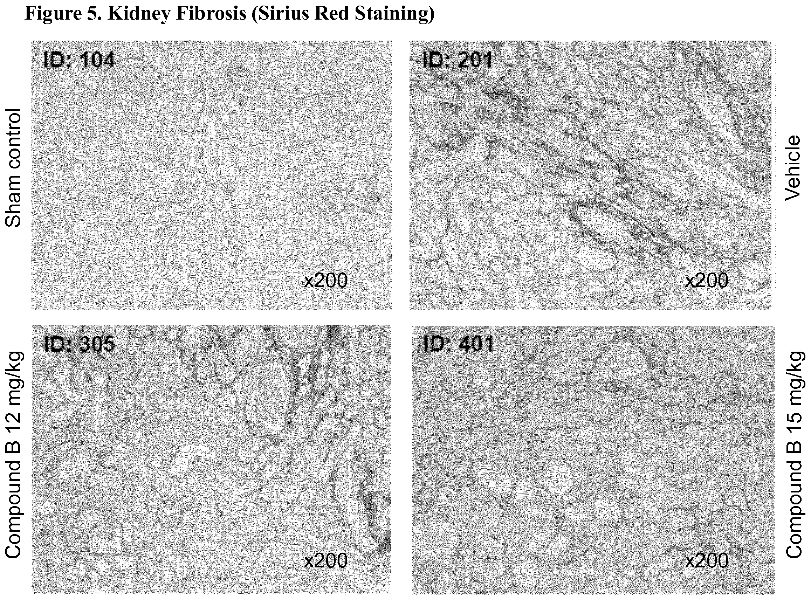

Figure 5 shows representative photomicrographs of UUO-induced kidney fibrosis revealed by Sirius red-staining of kidney sections. UUO induced significant kidney fibrosis (Vehicle group) in comparison to Sham control. Kidney fibrosis was significantly reduced in UUO animals treated with Compound B. x200: applied magnifications, ID: corresponds to sample number.

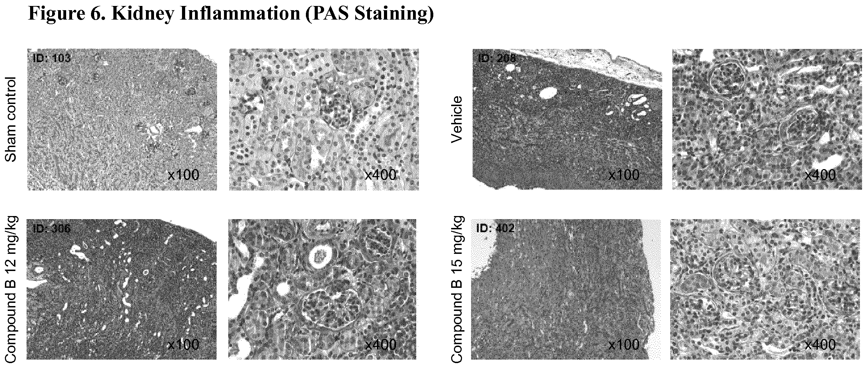

Figure 6 shows representative photomicrographs of UUO-induced kidney inflammation revealed by PAS-stained kidney sections. UUO induced significant kidney inflammation (Vehicle group) in comparison to Sham control. Kidney inflammation was reduced in UUO animals treated with Compound B, especially at the high dose group (15 mg/kg). x100, x400: different magnifications of the same sample. ID: corresponds to sample number.

### DETAILED DESCRIPTION OF THE INVENTION

The instant disclosure provides methods for preventing, ameliorating, treating, or even reducing symptoms of a fibrotic disorder or fibrotic disease. As used herein the term "treating or preventing" may be understood to relate to treating or preventing the disease itself or treating or preventing symptoms associated with the disease, including reduction in the disease and/or symptoms and/or slowing the progression of the disease and/or symptoms. The term fibrosis describes the development of fibrous connective tissue as a reparative response to injury or damage. Repair of damaged tissues is a fundamental biological process that allows the ordered replacement of dead or injured cells during an inflammatory response, a mechanism that is crucial for survival. The repair process typically involves two distinct stages: a regenerative phase, in which injured cells are replaced by cells of the same type and there is no lasting evidence of damage; and a phase known as fibroplasia or fibrosis, in which connective tissue replaces normal parenchymal tissue. In most cases, both stages are required to slow or reverse the damage caused by an injurious agent. However, although initially beneficial, the healing process can become pathogenic if it continues unchecked, leading to considerable tissue remodelling and the formation of permanent scar tissue. Fibrotic scarring is often defined as a wound-healing response that has gone awry.

An injury is an event that damages tissue and initiates the wound healing process. After injury, both mechanical (i.e. extracellular stress caused by disruption of the extracellular matrix, ECM) and chemical signals (e.g. inflammatory mediators like TGF.beta.) activate fibroblastic cells to increase production of extra cellular matrix (ECM) components, which begins the process of fibroblast differentiation into myofibroblasts (Tomasek et al., Nat. Rev. Mol. Cell Biol. 3:349, 2002; Werner et al., Physiol. Rev. 83:835, 2003). Depending on the type of tissue being remodeled, the fibroblasts that differentiate may come from different sources, including locally present fibroblasts, pericytes, smooth muscle cells, fibrocytes from bone marrow, and from epithelial-mesenchymal transition (EMT) (Hinz et al., Am. J. Pathol. 170:1807, 2007). Wound healing is seen as complete when the newly formed, crosslinked ECM takes over the mechanical load, which is a signal to myofibroblasts to undergo apoptosis (Tomasek et al., 2002; Carlson et al., J. Surg. Res. 110:304, 2003).

If the injury is severe or repetitive, or if the wound-healing process is dysregulated, fibrosis becomes pathogenic, resulting in permanent scarring or hardening of the tissue, organ malfunction or failure, and ultimately death. For example, idiopathic pulmonary fibrosis (IPF) is not completely understood but is seen as a progressive and fatal lung disease that has few effective treatments other than lung transplantation (Mason et al., Ann. Thorac. Surg. 84:1121-8, 2007). Median survival of five years after diagnosis is less than 20%. Most forms of interstitial lung diseases and other forms of pulmonary fibrosis are characterized by fibrotic lesions, progressive distortion of alveolar architecture occurs and replacement with fibrotic or scar tissues with excess ECM deposition (American Thoracic Society, Am. J. Respir. Crit. Care Med. 161:646, 2000; Noble et al., Clin. Chest Med. 25:749, 2004; Selman et al., Ann. Intern, Med. 134:136, 2001). This can result in progressive dyspnea and loss of lung function. A hallmark morphological lesion is spatial and temporal heterogeneity incorporating areas of normal lung being directly adjacent to areas of fully established fibrosis, microscopic honeycombing, and areas of evolving fibrosis containing collagen-producing fibroblasts/myofibroblasts, often referred to as "fibrotic foci."

The term "fibrotic disorder" or "fibrotic disease" (which are used interchangeably herein) refers to a medical condition featuring progressive and/or irreversible fibrosis, wherein excessive deposition of extracellular matrix occurs in and around inflamed or damaged tissue. In certain embodiments, a fibrotic disorder or disease is associated with the persistent presence of myofibroblasts in and around fibrotic foci or lesions. Excessive and persistent fibrosis can progressively remodel and destroy normal tissue, which may lead to dysfunction and failure of affected organs, and ultimately death. A fibrotic disorder may affect any tissue in the body and is generally initiated by an injury and the transdifferentiation of fibroblasts into myofibroblasts.

As used herein, "injury" refers to an event that damages tissue and initiates fibrosis. An injury may be caused by an external factor, such as mechanical insult (e.g., cut, surgery), exposure to radiation, chemicals (e.g., chemotherapy, toxins, irritants, smoke), or infectious agent (e.g., bacteria, virus, or parasite). An injury may be caused by, for example, chronic autoimmune inflammation, allergic response, HLA mismatching (e.g., transplant recipients), or ischemia (i.e., an "ischemic event" or "ischemia" refers to an injury that restricts in blood supply to a tissue, resulting in damage to or dysfunction of tissue, which may be caused by problems with blood vessels, atherosclerosis, thrombosis or embolism, and may affect a variety of tissues and organs; an ischemic event may include, for example, a myocardial infarction, stroke, organ or tissue transplant, or renal artery stenosis). In certain embodiments, an injury leading to a fibrotic disorder may be of unknown etiology (i.e., idiopathic).

Non-limiting examples of fibrotic disorders or fibrotic diseases include renal (kidney) fibrosis, pulmonary fibrosis, such as idiopathic pulmonary fibrosis, cystic fibrosis, liver fibrosis (e.g., cirrhosis), cardiac fibrosis, endomyocardial fibrosis, vascular fibrosis (e.g., atherosclerosis, stenosis, restenosis), atrial fibrosis, mediastinal fibrosis, myelofibrosis, retroperitoneal fibrosis, progressive massive fibrosis (e.g., lungs),, nephrogenic systemic fibrosis, Crohn's disease, hypertrophic scarring, keloid, scleroderma, systemic sclerosis (e.g., skin, lungs), athrofibrosis (e.g., knee, shoulder, other joints), Peyronie's disease, Dupuytren's contracture, adhesive capsulitis, organ transplant associated fibrosis, ischemia associated fibrosis, or the like.

In one aspect not according to the invention, the disclosure relates to the treatment, prevention or reduction of symptoms associated with renal (kidney) fibrosis. Reference to "renal fibrosis" or "kidney fibrosis" (the two terms are used interchangeably herein) means a progressive fibrotic manifestation of various diseases which may lead to severe illness and even death. For example, chronic kidney disease (CKD) can present in some patients with a potentially life-threatening fibrotic phenotype. This condition (CKD with fibrosis) may result from a variety of other serious indications such as diabetic nephropathy, hypertension, glomerulonephritis (GN) and polycystic disease. Without wishing to be bound by theory, it is believed that prior therapies intended to treat these diseases are often inadequate to halt the development or progression of fibrosis leading to severe illness and death in some cases. The present disclosure addresses this problem, for example, by providing a method that focuses on the development and/or progression of fibrosis in a patient that has, is suspected of having or is at risk of developing CKD, diabetic neuropathy, hypertension, glomerulonephritis (GN) and/or a polycystic disease.

In an embodiment, the invention relates to the treatment of a pulmonary fibrotic disorder. Reference to "pulmonary fibrotic disorder" means diseases or disorders characterized by fibrotic hypertrophy or fibrosis of lung tissue. Exemplary pulmonary fibrotic disorders include pulmonary fibrosis, idiopathic pulmonary fibrosis, interstitial lung disease, interstitial pulmonary fibrosis, chronic interstitial pneumonitis, Hamman-Rich Syndrome, usual interstitial pneumonitis (UIP), fibrosing alveolitis, pulmonary sarcoidosis, progressive massive fibrosis (e.g., lungs), systemic sclerosis (e.g., lungs), lung transplant associated fibrosis, or the like.

An aspect not according to the invention involves the use of compounds of formula (I) or salts thereof in the treatment or prevention of fibrotic disorders. In the compound of formula (I)

R-L-CO-X (I)

R is a C₁₀₋₂₄ unsaturated hydrocarbon group optionally interrupted by one or more heteroatoms or groups of heteroatoms selected from S, O, N, SO, SO₂, said hydrocarbon group comprising at least 4 non-conjugated double bonds;
L is a linking group forming a bridge of 1 to 5 atoms between the R group and the carbonyl CO wherein L comprises at least one heteroatom in the backbone of the linking group; and
X is an electron withdrawing group; or a salt thereof.

The group R preferably comprises 5 to 9 double bonds, preferably 5 or 8 double bonds, e.g. 5 to 7 double bonds such as 5 or 6 double bonds. These bonds should be non-conjugated. It is also preferred if the double bonds do not conjugate with the carbonyl functionality.

The double bonds present in the group R may be in the cis or trans configuration however, it is preferred if the majority of the double bonds present (i.e. at least 50%) are in the cis configuration. In further advantageous embodiments all the double bonds in the group R are in the cis configuration or all double bonds are in the cis configuration except the double bond nearest the carbonyl group which may be in the trans configuration.

The group R may have between 10 and 24 carbon atoms, preferably 12 to 20 carbon atoms, especially 17 to 19 carbon atoms.

Whilst the R group can be interrupted by at least one heteroatom or group of heteroatoms, this is not preferred and the R group backbone preferably contains only carbon atoms.

The R group may carry up to three substituents, e.g. selected from halo, C₁₋₆alkyl e.g. methyl, or C₁₋₆ alkoxy. If present, the substituents are preferably non-polar, and small, e.g. a methyl group. It is preferred however, if the R group remains unsubstituted.

The R group is preferably an alkylene group.

The R group is preferably linear. It preferably derives from a natural source such as a long chain fatty acid or ester. In particular, the R group may derive from AA, EPA or DHA.

Thus, viewed from another aspect not according to the invention, the disclosure employs a compound of formula (I')

R-L-CO-X (I')

wherein R is a C₁₀₋₂₄ unsubstituted unsaturated alkylene group said group comprising at least 4 non-conjugated double bonds;
L is a linking group forming a bridge of 1 to 5 atoms between the R group and the carbonyl CO wherein L comprises at least one heteroatom in the backbone of the linking group; and
X is an electron withdrawing group; or a salt thereof.

Ideally R is linear. R is therefore preferably an unsaturated C₁₀₋₂₄ polyalkylene chain.

The linking group L provides a bridging group of 1 to 5 backbone atoms, preferably 2 to 4 backbone atoms between the R group and the carbonyl, such as 2 atoms. The atoms in the backbone of the linker may be carbon and/or be heteroatoms such as N, O, S, SO, SO₂. The atoms should not form part of a ring and the backbone atoms of the linking group can be substituted with side chains, e.g. with groups such as C₁₋₆ alkyl, oxo, alkoxy, or halo.

Preferred components of the linking group are -CH₂-, -CH(C₁₋₆alkyl)-, -N(C₁₋₆alkyl)-, -NH-, -S-, -O-, -CH=CH-, -CO-, -SO-, -SO₂- which can be combined with each other in any (chemically meaningful) order to form the linking group. Thus, by using two methylene groups and an -S- group the linker -SCH₂CH₂- is formed. It will be appreciated that at least one component of the linker provides a heteroatom in the backbone.

The linking group L contains at least one heteroatom in the backbone. It is also preferred if the first backbone atom of the linking group attached to the R group is a heteroatom or group of heteroatoms.

It is highly preferred if the linking group L contains at least one -CH₂- link in the backbone. Ideally the atoms of the linking group adjacent the carbonyl are -CH₂-.

It is preferred that the group R or the group L (depending on the size of the L group) provides a heteroatom or group of heteroatoms positioned α, β, γ, or δ to the carbonyl, preferably β or γ to the carbonyl. Preferably the heteroatom is O, N or S or a sulphur derivative such as SO.

Highly preferred linking groups L therefore are -NH₂CH₂, -NH(Me)CH₂-, -SCH₂, -SOCH₂-, or -COCH₂-

The linking group should not comprise a ring.

Highly preferred linking groups L are SCH₂, NHCH₂, and N(Me)CH₂ .

Viewed from another aspect not according to the invention, the disclosure employs a compound of formula (II)

R-L-CO-X (II)

wherein R is a linear C₁₀₋₂₄ unsubstituted unsaturated alkylene group said group comprising at least 4 non-conjugated double bonds;
L is -SCH₂-, -OCH₂, -SOCH₂, or -SO₂CH₂-; and
X is an electron withdrawing group or a salt thereof.

The group X is an electron withdrawing group. Suitable groups in this regard include O-C₁₋₆ alkyl, CN, OCO₂-C₁₋₆ alkyl, phenyl, CHal₃, CHal₂H, CHalH₂ wherein Hal represents a halogen, e.g. fluorine, chlorine, bromine or iodine, preferably fluorine.

In a preferred embodiment the electron withdrawing group is CHal₃, especially CF₃.

Thus, preferred compounds of formula (I) are those of formula (III) (not according to the invention)

R-Y1-Y2-CO-X (III)

wherein R is a C₁₀₋₂₄ unsubstituted unsaturated alkylene group said group comprising at least 4 non-conjugated double bonds;
X is as hereinbefore defined;
Y1 is selected from O, S, NH, N(C₁₋₆-alkyl), SO or SO₂ and
Y2 is (CH₂)ₙ or CH(C₁₋₆ alkyl); or
where n is 1 to 3, preferably 1.

According to the present invention, the compounds are those of formula (IV)

R-Y1-CH₂-CO-X (IV)

wherein R is a linear C₁₀₋₂₄ unsubstituted unsaturated alkylene group said group comprising at least 4 non-conjugated double bonds;
X is CF₃; and
Y1 is selected from O, S, SO or SO₂.

Highly preferred compounds for use in the invention are depicted below. 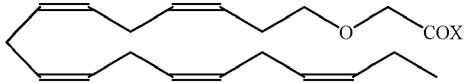 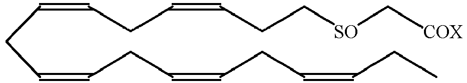 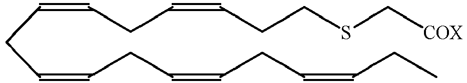 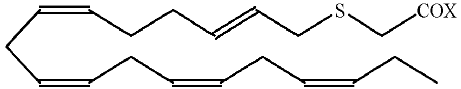 where X is CF₃.

The following compounds are highly preferred for use in the invention: 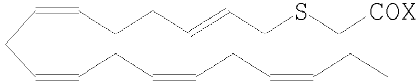 X= CF₃ = Compound A 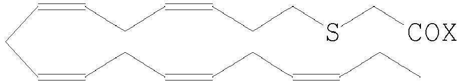 X= CF₃ = Compound B

According to the invention compound A or B are for use in the treatment or prevention of pulmonary or liver fibrosis.

Where possible, the compounds of the invention can be administered in salt, hydrate or solvate form, especially salt form.

Typically, a pharmaceutical acceptable salt may be readily prepared by using a desired acid. The salt may precipitate from solution and be collected by filtration or may be recovered by evaporation of the solvent. For example, an aqueous solution of an acid such as hydrochloric acid may be added to an aqueous suspension of a compound of formula (I) and the resulting mixture evaporated to dryness (lyophilised) to obtain the acid addition salt as a solid. Alternatively, a compound of formula (I) may be dissolved in a suitable solvent and the acid may be added in the same solvent or another suitable solvent. The resulting acid addition salt may then be precipitated directly, or by addition of a less polar solvent such as diisopropyl ether or hexane, and isolated by filtration.

Suitable addition salts are formed from inorganic or organic acids which form non-toxic salts and examples are hydrochloride, hydrobromide, hydroiodide, sulphate, bisulphate, nitrate, phosphate, hydrogen phosphate, acetate, trifluoroacetate, maleate, malate, fumarate, lactate, tartrate, citrate, formate, gluconate, succinate, pyruvate, oxalate, oxaloacetate, trifluoroacetate, saccharate, benzoate, alkyl or aryl sulphonates (e.g. methanesulphonate, ethanesulphonate, benzenesulphonate or p-toluenesulphonate) and isethionate. Representative examples include trifluoroacetate and formate salts, for example the bis or tris trifluoroacetate salts and the mono or diformate salts, in particular the tris or bis trifluoroacetate salt and the monoformate salt.

Compounds of formula (IV) may be manufactured using known chemical synthetic routes. It is convenient to begin synthesis from the commercially available compounds arachidonic acid (AA), EPA (all-Z-eicosa-5,8,11,14,17-pentaenoic acid) or DHA (all-Z-docosa-4,7,10,13,16,19-hexaenoic acid). Conversion of the acid functionality of these compounds into, for example a -COCF₃ group can be achieved readily, e.g. by converting the carboxylic acid into its corresponding acid chloride and reacting the same with trifluoroacetic anhydride in the presence of pyridine.

Introduction of a heteroatom into the carbon chain is also achieved readily. Conveniently, for example, the starting acid is reduced to an alcohol and, if required, converted to the corresponding thiol. The nucleophilic thiol may then be reacted with a group such as BrCH₂COCF₃ thereby introducing the carbonyl and electron withdrawing species. Complete synthetic protocols may be found in J. Chem. Soc., Perkin Trans 1, 2000, 2271-2276 or J. Immunol., 1998, 161, 3421.

The amount of the compounds of the invention in the composition will often be determined by the physician depending on the dosage required.

The composition of the invention is proposed for use in the treatment or prevention of fibrotic disorders, wherein the fibrotic disorder is a pulmonary fibrotic disorder or liver fibrosis.

By treating or treatment is meant at least one of:
(i). inhibiting the disease i.e. arresting, reducing or delaying the development of the disease or a relapse thereof or at least one clinical or subclinical symptom thereof, or
(ii). relieving or attenuating one or more of the clinical or subclinical symptoms of the disease.

By prevention is meant (i) preventing or delaying the appearance of clinical symptoms of the disease developing in a mammal.

The benefit to a subject to be treated is either statistically significant or at least perceptible to the patient or to the physician. In general a skilled man can appreciate when "treatment" occurs. It is particularly preferred if the composition of the invention are used therapeutically, i.e. to treat a condition which has manifested rather than prophylactically. It may be that the composition of the invention is more effective when used therapeutically than prophylactically.

The composition of the invention can be used on any animal subject, in particular a mammal and more particularly to a human or an animal serving as a model for a disease (e.g., mouse, monkey, etc.).

In order to treat a disease an effective amount of the active composition needs to be administered to a patient. A "therapeutically effective amount" means the amount of a composition that, when administered to an animal for treating a state, disorder or condition, is sufficient to effect such treatment. The "therapeutically effective amount" will vary depending on the composition , the disease and its severity and the age, weight, physical condition and responsiveness of the subject to be treated and will be ultimately at the discretion of the attendant doctor.

It may be that to treat fibrosis according to the invention that the composition of the invention has to be readministered at certain intervals. Suitable dosage regimes can be prescribed by a physician.

The composition of the invention typically comprises the active components in admixture with at least one pharmaceutically acceptable carrier selected with regard to the intended route of administration and standard pharmaceutical practice.

The term "carrier" refers to a diluent, excipient, and/or vehicle with which an active compound is administered. The pharmaceutical compositions of the invention may contain combinations of more than one carrier. Such pharmaceutical carriers are well known in the art. The pharmaceutical compositions may also comprise any suitable binder(s), lubricant(s), suspending agent(s), coating agent(s), and/or solubilizing agent(s) and so on. The compositions can also contain other active components, e.g. other drugs for the treatment of cancer.

It will be appreciated that pharmaceutical composition for use in accordance with the present invention may be in the form of oral, parenteral, transdermal, sublingual, topical, implant, nasal, or enterally administered (or other mucosally administered) suspensions, capsules or tablets, which may be formulated in conventional manner using one or more pharmaceutically acceptable carriers or excipients. The compositions of the invention could also be formulated as nanoparticle formulations.

However, for the treatment of fibrosis, the composition of the invention can be administered by a variety of routes such as orally or parenterally (e.g., subcutaneous, intramuscular or intravenous). For many invention embodiments subcutaneous administration will be preferred.. In embodiments in which compositions of the invention are administered orally, the composition may therefore be provided in the form of a tablet or solution for injection.

The pharmaceutical composition of the invention may contain from 0.01 to 99% weight - per volume of the active material. The therapeutic doses will generally be between about 10 and 2000 mg/day and preferably between about 30 and 1500 mg/day. Other ranges may be used, including, for example, 50-500 mg/day, 50-300 mg/day, 100-200 mg/day.

Administration may be once a day, twice a day, or more often, and may be decreased during a maintenance phase of the disease or disorder, e.g. once every second or third day instead of every day or twice a day. The dose and the administration frequency will depend on the clinical signs, which confirm maintenance of the remission phase, with the reduction or absence of at least one or more preferably more than one clinical signs of the acute phase known to the person skilled in the art.

Treatment according to the invention may be carried out in conjunction with other known treatments for the fibrotic disease in question. For example, patients with pulmonary fibrous may be administered oxygen. Treatment according to the invention may be carried out in other embodiments in conjunction with other known treatments in the same or different pharmaceutical compositions. Illustrative "combination agents" may include, among others: an immunosuppressive drug including, but not limited to, cyclosporine, azathioprine, cyclophosphamide, or mycophenolate mofetil; an anti-inflammatory drug including, but not limited to, corticosteroids (e.g. prednisone), a cytokine including but not limited to, interferon-alpha, interferon gamma, interleukin 12, a TNF-, CCR2-, CCR5- or VAP1-inhibitor; thalidomide; an anti-hypertensive agent including but not limited to ACE inhibitors, ARBs, renin inhibitors and mineralcorticoid receptor antagonists (e.g. captopril, ramipril, lisinopril, losartan, telmisartan, aliskiren, spironolactone, finerenone, CS-3150, MT-3995, eplerenone etc); a monoclonal antibody or other agent targeting, among others, CTGF, TGF-β, MCP-1, IL-4, and IL-13; a multiple receptor tyrosine kinase inhibitor including, but not limited to, nintedanib and the JNK (kinase) inhibitor tanzisertib (CC-930) or ruxolitinib (Jakavi™); an antioxidant such as, but not limited to, N-acetylcysteine, pirfenidone, vitamin E, S-adenosyl methionine, pyridorin, GKT137831, or penicillamine; an enzyme inhibitor including, but not limited, to Lysyloxidase-like-2 (LOXL2 enzyme); an integrin inhibitor such as, but not limited to, αᵥβ₆; a lipid receptor modulator or a hypolipidemic agent including, but not limited to, lysophosphatidic acid receptor antagonists, HMG-CoA reductase inhibitors, Stearoyl -CoA Desaturase inhibitors, PPAR inhibitors and thiazolindiones or cholesterol absorption inhibitors; an inhibitor affecting vasculature or angiogenesis including but not limited to ET_{A} inhibitors such as atrasentan, SGLT2 inhibitors such as canagliflozin; pomalidomide; an apoptosis inhibitor such as IDN-6556 or GS-4997; a PDE inhibitor such as CTP-499; a thrombomodulin inhibitor or a therapy based on stem cells (SCs) such as umbilical cord SCs, autologous SCs and bone marrow SCs;

The invention is described further below with reference to the following non-limiting examples and figures.

### Example 1 (Reference example)

Unilateral Ureteral Obstruction (UUO) is a well-validated rodent model of renal injury utilised to study the pathophysiology of renal interstitial fibrosis. The model is based on surgical ligation of a single (left or right) ureter under anaesthesia which triggers an increase in the hydrostatic pressure ensuing from the obstruction leading to progressive tubular cell death by apoptosis and necrosis, interstitial inflammatory infiltration, capillary rarefaction and progressive fibrosis with loss of renal parenchyma, myofibroblast activation and extracellular matrix deposition (reviewed in Dendooven A et al, Int. J. Exp. Path. (2011), 92, 202-210). As UUO-induced kidney fibrosis can be developed within 7 or 14-days post ureter ligation, this model is well suited to assess the anti-fibrotic effects of compounds administered to rodents within a reasonably short period of time (Eddy A et al, Pediatr Nephrol. 2012 Aug;27(8):1233-47).

### Test article & formulations

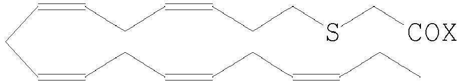

X= CF₃ = Compound B

Compound B was formulated for subcutaneous administration in a nano-emulsion containing 2 mg/ml Compound B, 2 mg/ml MCT oil (Lipoid), 2 mg/ml Polysorbate 80 (Fluka) and sodium citrate buffer 10 mM (pH 7). The final solution was sterilized by filtration and saturated with Argon 5.0 AGA prior to aliquoting in 10 ml tubes stored at - 20° C till further use. A separate Polysorbate 80- and sodium citrate buffer-based vehicle solution without Compound B and MCT oil was also prepared, sterilized, aliquoted and stored, serving as the subcutaneous vehicle control used in the UUO animal study.

### Animals

Seven-week-old female C57BL/6J mice were obtained from Japan SLC, Inc. (Japan). Animals were kept in an animal room facility at a temperature of 23 ± 2°C, humidity of 45 ± 10%, under a 12-hour light and dark cycle (light from 8:00 to 20:00). A high pressure of 20 ± 4 Pa was kept in the experimental room to prevent contamination. The animals were housed in TPX cages (CLEA Japan) and fed with a sterilized normal diet (CE-2; CLEA Japan, Japan) provided ad libitum, being placed in a metal lid on the top of the cage. Pure water was provided ad libitum from a water bottle equipped with a rubber stopper and a sipper tube.

### Experimental Design

29 female C57BL/6J mice were divided into groups and treated according to the design of Table 1 below (Day 0 to Day 13).

**Table 1. Groups and Treatments**

| Group | No. mice | Model | Test substance | Dose (mg/kg) | Volume (mL/kg) | Regimen | Sacrifice (Day) |
|---|---|---|---|---|---|---|---|
| 1 | 5 | Normal | - | - | - | - | 14 |
| 2 | 8 | UUO | Vehicle | - | 6 | Subcutaneous, daily, Day 0 - Day 13 | 14 |
| 3 | 8 | UUO | Compound B | 6 | 3 | Subcutaneous, daily Day 0 - Day 13 | 14 |
| 4 | 8 | UUO | Compound B | 12 | 6 | Subcutaneous, daily Day 0 - Day 13 | 14 |

### Induction of UUO

Mice under anesthesia (medetomidine, midazolam, butorphanol) were shaved on the left side of the abdomen. A vertical incision was made through the skin with a scalpel and the skin was retracted. A second incision was made through the peritoneum and that skin was also retracted to reveal the kidney. Using forceps the kidney was brought to the surface and the middle portion of the left ureter was ligated with 4-0 nylon surgical silk at two points. The ligated kidney was placed back into its correct anatomical position and sterile saline was added to replenish loss of fluid. The incisions are sutured and mice were caged individually for further experimentation.

### Group Treatments

Table 1 above summarizes the treatment schedules of the study. Compound B (at doses of 6 or 12 mg/kg) or vehicle were administered subcutaneously (SC) for 13 days daily following UUO (Groups 2-4). No UUO was conducted in animals of Group 1 which served as a normal/sham (no disease) group. Animals were sacrificed at Day 14.

### Group Assessments

The viability, clinical signs and behavior were monitored daily. Individual body weight was measured daily before the treatment. Mice were observed for significant clinical signs of toxicity, moribundity and mortality approximately 60 minutes after each administration. The animals were sacrificed by exsanguination through direct cardiac puncture under isoflurane anesthesia at Day 14 according to standard ethical procedures.

### Sample collection, assays and measurements

### Measurement of plasma biochemistry

For plasma biochemistry, non-fasting blood was collected in polypropylene tubes with anticoagulant (Novo-Heparin, Mochida Pharmaceutical Co. Ltd., Japan) and centrifuged at 1,000xg for 15 minutes at 4°C. The supernatant was collected and stored at -80°C until use. Plasma Blood Urea Nitrogen (BUN) was measured by FUJI DRI-CHEM 7000 (Fujifilm, Japan).

### Measurement of kidney biochemistry

To quantify kidney hydroxyproline content, frozen left kidney samples were processed by an alkaline-acid hydrolysis method as follows. Kidney samples were dissolved in 2N NaOH at 65°C, and autoclaved at 121°C for 20 minutes. The lysed samples (400 µL) were acid-hydrolyzed with 400 µL of 6N HCl at 121°C for 20 minutes, and neutralized with 400 µL of 4N NaOH containing 10 mg/mL activated carbon. AC buffer (2.2M acetic acid/0.48M citric acid, 400 µL) was added to the samples, followed by centrifugation to collect the supernatant. A standard curve of hydroxyproline was constructed with serial dilutions of trans-4-hydroxy-L-proline (Sigma-Aldrich, USA) starting at 16 µg/mL. The prepared samples and standards (each 400 µL) were mixed with 400 µL chloramine T solution (Wako Pure Chemical Industries, Japan) and incubated for 25 minutes at room temperature. The samples were then mixed with Ehrlich's solution (400 µL) and heated at 65°C for 20 minutes to develop the color. After samples were cooled on ice and centrifuged to remove precipitates, the optical density of each supernatant was measured at 560 nm. The concentrations of hydroxyproline were calculated from the hydroxyproline standard curve. Protein concentrations of kidney samples were determined using a BCA protein assay kit (Thermo Fisher Scientific, USA) and used to normalize the calculated hydroxyproline values. Kidney hydroxyproline contents were expressed as µg per mg protein.

### Histopathological analyses

For PAS staining, sections were cut from paraffin blocks and stained with Schiff's reagent (Wako Pure Chemical Industries) according to the manufacturer's instructions. To observe the tubular damages, bright field images in the corticomedullary region were captured using a digital camera (DFC295; Leica Microsystems, Germany) at 100- and 400-fold magnifications.

The kidney was fixed in 10% neutral buffered formalin (Wako Pure Chemical Industries) and embedded in paraffin. To visualize collagen deposition, kidney sections were stained using picro-Sirius red solution (Waldeck, Germany). For quantification of interstitial fibrosis area, bright field images in the corticomedullary region were captured using a digital camera (DFC295) at 200-fold magnification, and the positive areas in 5 fields/section were measured using ImageJ software (National Institute of Health, USA).

### Quantitative RT-PCR of Collagen Type 1 mRNA

Total RNA was extracted from kidney samples using RNAiso (Takara Bio, Japan) according to the manufacturer's instructions. One µg of RNA was reverse-transcribed using a reaction mixture containing 4.4 mM MgCl₂ (F. Hoffmann-La Roche, Switzerland), 40 U RNase inhibitor (Toyobo, Japan), 0.5 mM dNTP (Promega, USA), 6.28 µM random hexamer (Promega), 5 x first strand buffer (Promega), 10 mM dithiothreitol (Invitrogen, USA) and 200 U MMLV-RT (Invitrogen) in a final volume of 20 µL. The reaction was carried out for 1 hour at 37°C, followed by 5 minutes at 99°C. Real-time PCR was performed using real-time PCR DICE and SYBR premix Taq (Takara Bio). To calculate the relative mRNA expression level of collagen type I gene, its expression was normalized to that of the reference gene GAPDH. The following PCR-primer sets have been used:

### GAPDH:

Forward: 5'-TGTGTCCGTCGTGGATCTGA-3' (SEQ ID NO:1)
Reverse: 5'-TTGCTGTTGAAGTCGCAGGAG-3' (SEQ ID NO:2)

### Collagen Type 1

Forward: 5'-CCAACAAGCATGTCTGGTTAGGAG-3' (SEQ ID NO:3)
Reverse: 5'-GCAATGCTGTTCTTGCAGTGGTA-3' (SEQ ID NO:4)

### Statistical tests

Statistical analyses were performed using Bonferroni Multiple Comparison Test on GraphPad Prism 6 (GraphPad Software, USA). P values < 0.05 were considered statistically significant. A trend or tendency was assumed when a one-tailed t-test returned P values < 0.1. Results were expressed as mean ± SD.

### Results

### Biochemical analyses

No significant differences were observed in plasma Blood Urea Nitrogen (BUN) measured in plasma samples from different groups (not shown). For what concerns hydroxyproline, the Vehicle group showed a significant increase in kidney hydroxyproline content compared with the Sham control group (p<0.0001). Compound B reduced kidney hydroxyproline in dose dependent manner (Table 2).

**Table 2. Kidney Hydroxyproline results**

| **Parameter** | **Sham control** | **Vehicle** | **Compound B 6 mg/kg** | **Compound B 12 mg/kg** |
|---|---|---|---|---|
| (mean ± SD) | (n=5) | (n=8) | (n=8) | (n=8) |
| Kidney hydroxyproline (mg/mg total protein) | 3.88 ± 0.31 | 18.50 ± 6 .66 | 16.24 ± 3.49 | 14.80 ± 3.48 |

### Histological analyses

### Sirius red staining

Representative photomicrographs of Sirius red-stained kidney sections are shown in

Figure 1. Quantification of interstitial fibrosis area (Table 3) showed a significant increase in the fibrosis area (Sirius red-positive area) of the Vehicle group compared with the Sham control group (p<0.0001). The Compound B high dose group showed a significant decrease in the fibrosis area compared with the Vehicle group (p<0.05). The Compound B low dose group also tended to decrease the fibrosis area compared with the Vehicle group (Table 3).

**Table 3. Fibrosis area (Sirius Red staining)**

| **Parameter** | **Sham control** | **Vehicle** | **Compound B 6 mg/kg** | **Compound B 12 mg/kg** |
|---|---|---|---|---|
| (mean ± SD) | (n=5) | (n=8) | (n=8) | (n=8) |
| Sirius red-positive area (%) | 1.27 ± 0.69 | 5.90 ± 1.86 | 4.88 ± 0.92 | 4.08 ± 1.58 |

### PAS staining

Representative photomicrographs of PAS-stained (Periodic acid-Schiff) kidney sections are shown in Figure 2. Kidney sections from the Vehicle group exhibited the inflammatory cell infiltration, severe tubular dilation, atrophy and PAS-positive cast formation in both the cortical and medullar regions. The inflammatory cell infiltration was reduced upon Compound B treatment especially at the high dose treatment group. No obvious differences were found in the tubular damages between the Vehicle group and the Compound B treatment groups.

### Body Weights, Toxicity of Compounds, and Mortality

No significant weight changes were observed during the treatment periods. There were no dead animals in the study and none of the animals showed deterioration in general condition. These results indicated lack of toxicity of Compound B treatments.

### Collagen Type 1 mRNA Expression

The Vehicle group showed a significant up-regulation in Collagen Type 1 mRNA expression level compared with the Sham control group (p<0.0001). Compound B at the high dose showed significant down-regulation in Collagen Type 1 mRNA expression levels compared to the Vehicle group (p<0.0001). The low dose group of Compound B also tended to down-regulate Collagen Type 1 mRNA expression level compared with the Vehicle group (Table 4).

**Table 4. Collagen Type 1 mRNA expression (qRT-PCR)**

| **Parameter** | **Sham control** | **Vehicle** | **Compound B 6 mg/kg** | **Compound B 12 mg/kg** |
|---|---|---|---|---|
| (mean ± SD) | (n=5) | (n=8) | (n=8) | (n=8) |
| Collagen Type 1 mRNA | 1.00 ± 0.24 | 96.68 ± 23.76 | 76.94 ± 18.71 | 48.71 ± 17.97 |

As can be seen from this example, UUO-induced significant kidney fibrosis 14-days post UUO in C57B mice. Treatment with Compound B showed a significant decrease in the fibrosis area in a dose-dependent manner. Compound B also reduced kidney hydroxyproline content and collagen Type 1 mRNA expression. PAS staining showed that inflammatory cell infiltration in the Compound B treatment group was reduced in comparison to the Vehicle group. These results showed that Compound B possesses anti-fibrotic and anti-inflammatory effects in the UUO model.

### EXAMPLE 2: Compound B inhibits TGF-β1 induced expression of fibrosis-markers and PGE2.

Fibrosis is understood to be a result of tissue injury and chronic inflammation. TGF-β1 is reported to be a central regulator of connective tissue and scar tissue deposition in fibrosis, ultimately impairing or destroying the function of an organ, such as the kidneys, lungs or liver. The following data shows, among other things, that a cPLA2α inhibitor efficiently and dose-dependently reduces the transcriptional levels of key fibrotic factors and reduces the production of the proinflammatory eicosanoid PGE2 in several cellular models for fibrosis.

Three different cell lines were treated with TGF-β1 to induce markers of fibrosis. The anti-fibrotic effects of Compound B were measured by mRNA expression of key fibrosis markers. An effect of Compound B on the levels of the metabolite PGE2 is also shown in one of the cell lines.

The following materials and methods were used as needed.

### Cell culture

NRK-49F (normal rat kidney fibroblasts, ATCC® CRL-1570™) were maintained in DMEM with 4500 mg/L glucose, 5% FBS, L-glutamine, and gentamicin. For experiments, 3 x 10^5 cells/well were seeded in 6-well plates. After three days, post-confluent cells were serum starved for 24h. They were then pre-incubated with inhibitors for 90 min, before they were treated with 10 ng/ml TGF-β1 for 24 h (mRNA-expression) or 72 h (PGE2).

MRC-5 (human lung fibroblast, ATCC® CCL-171™) were maintained in MEM with 10% FBS, L-glutamine, and gentamicin. For experiments, 1 x 10^5 cells/well were seeded in 6-well plates. After two days, pre-confluent cells were serum starved for 24h. They were then pre-incubated with inhibitors for 90 min, before they were treated with 2 ng/ml TGF-β1 for 24 h.

RMC (rat mesangial cells, ATCC® CRL-2573™) were maintained in DMEM with 4500 mg/L glucose (Sigma), 15% FBS, L-glutamine, and 0.4 mg/ml G418. For experiments, 3 x 10^5 cells/well were seeded in 6-well plates. After five days, post-confluent cells were serum starved for 24h. They were then pre-incubated with inhibitors for 90 min, before they were treated with 5 ng/ml TGF-β1 for 24 h.

### qRT-PCR

Total RNA was isolated using the RNeasy Mini Kit (Qiagen). cDNA synthesis was performed with 1 µg total RNA in 20 µl reaction of QuantiTect Reverse Transcription Kit (Qiagen). After synthesis, cDNA was diluted 1:6 with RNase-free water. qRT-PCR was performed with LightCycler 480 SYBR Green I Master (Roche), and the qRT-PCR analyses were carried out using the Lightcycler 96 system (Roche). Primer sequences are listed in Table 5.

**Table 5: Primer sequences**

| Oligo name | Forward (5'-3') | Reverse (5'-3') |
|---|---|---|
| Rat Acta2 | GCCATCAGGAACCTCGAGAA (SEQ ID NO:5) | GGAGCATCATCACCAGCAAAG (SEQ ID NO:6) |
| Rat Ptgs2 | CTCATACTGATAGGAGAGACG (SEQ ID NO:7) | TCGAACTTGAGTTTGAAGTG (SEQ ID NO:8) |
| Rat Colla2 | AGTGGAAGAGCGATTACTAC (SEQ ID NO:9) | ATTGATGGTCTCTCCTAACC (SEQ ID NO:10) |
| Rat Col3a1 | AGTGGCCATAATGGGGAACG (SEQ ID NO:11) | CAGGGTTTCCATCCCTTCCG (SEQ ID NO:12) |
| Rat Fn1 | GGACACTATGCGGGTCACTTG G (SEQ ID NO:13) | TGCTGTTCGTACACGCTGGAGA (SEQ ID NO:14) |
| Rat 18S | CGATTCCGTGGGTGGTGGTG (SEQ ID NO:15) | CATGCCAGAGTCTCGTTCGTTAT C (SEQ ID NO:16) |
| Human ACTA2 | AGATCAAGATCATTGCCCC (SEQ ID NO:17) | TTCATCGTATTCCTGTTTGC (SEQ ID NO:18) |
| Human PTGS2 | AAGCAGGCTAATACTGATAGG (SEQ ID NO:19) | TGTTGAAAAGTAGTTCTGGG (SEQ ID NO:20) |
| Human COL4A 1 | AAAGGGAGATCAAGGGATAG (SEQ ID NO:21) | TCACCTTTTTCTCCAGGTAG (SEQ ID NO:22) |
| Human COL3A 1 | ATTCACCTACACAGTTCTGG (SEQ ID NO:23) | TGCGTGTTCGATATTCAAAG (SEQ ID NO:24) |
| Human CTGF | TTAAGAAGGGCAAAAAGTGC (SEQ ID NO:25) | CATACTCCACAGAATTTAGCTC (SEQ ID NO:26) |
| Human RPS18 | CAGAAGGATGTAAAGGATGG (SEQ ID NO:27) | TATTTCTTCTTGGACACACC (SEQ ID NO:28) |

### Enzyme linked immunoassay detection of PGE2

Cell supernatant samples were analyzed by enzyme-linked immunosorbent assay (EIA) for PGE2 (Cayman #514010) according to the kit protocol. Cell supernatants were assayed undiluted. Supernatants were hybridized with over-night incubation, enzymatic conversion of substrate were read at OD420 nm. Data were processed using a 4-parameter logistic fit model.

Transforming growth factor β1 (TGF-β1) has been reported to be a major driver of fibrosis, and induces the differentiation of fibroblasts to myofibroblasts. The differentiation into myofibroblasts is said to be characterized by de novo synthesis of α-smooth muscle actin (a-SMA). As shown in Figure 3 a-c, TGF-β1 is a powerful inducer of *α-SMA* mRNA in both fibroblast cell lines tested and in the mesangial cell line. The cPLA2α inhibitor Compound B (5 µM) inhibits TGF-β1 induced expression of *α-SMA* mRNA in NRK-49F cells by 50 % (Figure 3a). In MRC-5, Compound B is most efficient, decreasing *α-SM A* mRNA expression by 76 % (Figure 3b). Also in the mesangial cell line, RMC, Compound B reduces *α-SMA* mRNA expression around 35 %, however not significantly (Figure 3c).

Activated myofibroblasts and mesangial cells are said to both contribute to a significant increase in ECM production. Collagens are the most abundant components of the ECM, and TGF-β1 induces several collagens in NRK-49F and MRC-5. Compound B treatment dose-dependently reduced the collagen levels. In NRK-49F cells, 10 µM Compound B reduce the levels of *Col1a2* by 34 % (Figure 3d) and *Col3a1* by 46 % (Figure 3f). In MRC-5 cells, 5 µM Compound B reduce the levels of *Col4a1* by 50 % (Figure 3e) and *Col3a1* by 38 % (Figure 3g).

Fibronectin is thought to play a role of "master organizer" in matrix assembly as it forms a bridge between cell surface receptors and compounds like collagens and proteoglycans in the ECM [Halper and Kjaer, 2014]. In NRK-49F cells, a dose-dependent reduction of Fibronectin levels was observed in response to Compound B. Treatment with 10 µM Compound B led to 30 % reduction in *Fibronectin* levels (Figure 3h). Furthermore, we show that TGF-β1 treatment of MRC-5 cells induces mRNA expression of the matricellular protein connective tissue growth factor (CTGF), which regulate the production of ECM and could contribute to fibrosis [Rayego-Mateos, 2018]. Treatment with 5 µM Compound B significantly reduces the level of *CTGF* mRNA by 42 % (Figure 3i).

cPLA2α is thought to be a key player regulating arachidonic acid (AA) release for eicosanoid biosynthesis [Hao, 2007]. The cyclooxygenase (COX) enzymatic pathway converts AA to the biologically active, proinflammatory, eicosanoid prostaglandin E2 (PGE2). TGF-β1 induces mRNA expression of *Ptgs2* (encoding the COX2 protein) in all three cell lines. 5 µM Compound B reduces TGF-β1 induced *Ptgs2* expression by 44 % in NRK-49F cells (Figure 3j). In MRC-5 cells, 5 µM Compound B reduces *Ptgs2* expression by 44 % and 58 %, respectively (Figure 3k). Also in RMC, 5 µM Compound B seem to reduce *Ptgs2* mRNA levels (Figure 3l). In line with the observed reduced expression of *Ptgs2,* we show that TGF-β1 induced PGE2 production is dose-dependently reduced in the presence of Compound B (Figure 4). 2 µM Compound B reduces PGE2 levels by 26 % and 5 µM Compound B reduces the levels by 58 %.

In Figure 3 column 1 shows post-confluent NRK-49F cells pre-treated with inhibitors for 90 min, before treatment with TGF-β1 (10 ng/ml, 24 h). Results shown are average of three biologically independent experiments.

Column 2 shows pre-confluent MRC-5 cells pre-treated with inhibitors for 90 min, before treatment with TGF-β1 (2 ng/ml, 24 h). Results shown are average of three biologically independent experiments.

Column 3 shows post-confluent RMC cells pre-treated with inhibitors for 90 min, before treatment with TGF-β1 (5 ng/ml, 8 h (a-SMA) or 24 h (Ptgs2)). Results from one experiment is shown.

In figure 4, post-confluent NRK-49F cells pre-treated with inhibitors for 90 min, before treatment with 10 ng/ml TGF-β1 for 72 h. Results shown are the average of two biologically independent experiments.

In the figures the following abbreviations are used:
TGF-β1, transforming growth factor β1;
α-SMA, α-smooth muscle actin;
Ptgs2, prostaglandin endoperoxide synthase 2/cyclooxygenase 2;
Col1a2, collagen 1a2;
Col3a1, collagen 3a1;
Col4a1, collagen 4a1;
CTGF, connective tissue growth factor.

### EXAMPLE 3 (Reference example)

### : UUO Model Repeated With Higher Doses of Compound B Shows Reduction in Kidney Fibrosis

The UUO model experiment described in Example 1 was repeated except as indicated below. Materials and methods not described below can be found elsewhere in this specification.

Similar anti-fibrotic and anti-inflammatory results were obtained in this UUO experiment conducted along lines of Example 1 except with 12 mg/kg and 15 mg/kg of Compound B. Briefly, 29 female C57BL/6J mice were divided into groups and treated according to the design of Table 1 with the sole differences being that Compound B was dosed at 12 and 15 mg/kg in dose volumes of 6 and 7.5 ml/kg, respectively (First experiment: 6 and 12 mg/kg of Compound B, in dose volumes of 3 and 6 ml/kg, respectively). Results from this experiment are presented in Tables 6 and 7 below.

**Table 6. Kidney Hydroxyproline results**

| **Parameter** | **Sham control** | **Vehicle** | Compound **B 12 mg/kg** | Compound **B 15 mg/kg** |
|---|---|---|---|---|
| (mean ± SD) | (n=5) | (n=8) | (n=8) | (n=8) |
| Kidney hydroxyproline (mg/mg total protein) | 4.53 ± 0.42 | 39.92 ± 12.50 | 30.50 ± 9.99 | 37.42±12.96 |

**Table 7. Fibrosis area (Sirius Red staining)**

| **Parameter** | **Sham control** | **Vehicle** | Compound **B 12 mg/kg** | Compound B **15 mg/kg** |
|---|---|---|---|---|
| (mean ± SD) | (n=5) | (n=8) | (n=8) | (n=8) |
| Sirius red-positive area (%) | 0.69 ± 0.20 | 6.80 ± 1.86 | 4.42 ± 1.43 | 3.90 ± 0.97 |

Representative photomicrographs of Sirius red-stained kidney sections are shown in Figure 5 whereas representative photomicrographs of PAS-stained (Periodic acid-Schiff) kidney sections are shown in Figure 6. Overall, the results of the experiment show at least the following:
For hydroxyproline (Table 6), the Vehicle group showed a significant increase in kidney hydroxyproline content compared with the Sham control group (p<0.0001). Compound B seemed to reduce kidney hydroxyproline especially at the low dose group (12 mg/kg). Comparing the Vehicle group with the Sham control group, a significant increase in the fibrosis area (Sirius red-positive area) was observed (p<0.0001). The Compound B dosed at 15 mg/kg showed a significant decrease in the fibrosis area compared with the Vehicle group (p<0.01). The Compound B dosed at 12 mg/kg also decreased the fibrosis area in a significant manner in comparison to the Vehicle group (p<0.05).

Kidney sections from the Vehicle group exhibited the inflammatory cell infiltration, severe tubular dilation, atrophy and PAS-positive cast formation in both the cortical and medullar regions. The inflammatory cell infiltration was reduced upon Compound B treatment at 15 mg/kg. No obvious differences were found in the tubular damages between the Vehicle group and the Compound B 12 mg/kg groups.

Finally, and in comparable fashion to the UUO experiment described in Example 1, no significant weight changes were observed during the treatment periods. There were no dead animals in the study and none of the animals showed deterioration in general condition. These results confirmed the lack of toxicity of Compound B treatment.

The UUO-induced significant kidney fibrosis 14-days post UUO in C57B mice. Treatment with Compound B showed a significant decrease in the fibrosis area. PAS staining showed that inflammatory cell infiltration in the high Compound B treatment group was reduced in comparison to the Vehicle group. These results showed that Compound B possesses anti-fibrotic and anti-inflammatory effects in the UUO model.

### Example 4: Compound B Shows Anti-fibrotic Activity In Bleomycin Model of Idiopathic Lung Fibrosis.

The bleomycin model of idiopathic fibrosis (IPF) is a well-characterized and currently an extensively used animal model of Interstitial lung disease (ILD) due to its: i) ability to reproduce many aspects of the disease, ii) good reproducibility, and iii) ease of induction. Overall, studies using the bleomycin model have identified many of the cellular and molecular mechanisms now recognized as being important in pathogenesis of IPF, as well as novel therapies for ILDs. The model is based on intratracheal administration of bleomycin in mice that results in alveolar epithelial damage, alveolar inflammation and fibrosis within a period of 21 days. Consequently, the model allows therapeutic testing of novel agents within a reasonably short period of time (Liu T eta al, Methods Mol Biol. 2017; 1627:27-42).

### Test article & formulations

Compound B (Synthetica A/S, Norway) was formulated for subcutaneous administration in a nano-emulsion containing 2 mg/ml Compound B, 2 mg/ml MCT oil (Lipoid), 2 mg/ml Polysorbate 80 (Fluka) and sodium citrate buffer 10 mM (pH 7). The final solution was sterilized by filtration and saturated with Argon 5.0 AGA prior to aliquoting in 10 ml tubes stored at -20° C till further use. A separate Polysorbate 80- and sodium citrate buffer-based vehicle solution without Compound B and MCT oil was also prepared, sterilized, aliquoted and stored, serving as the subcutaneous vehicle control used in the UUO animal study. Nintedanib (approved and commercialized for IPF under the brand names Ofev/Vargatef) was used as a positive control. The compound was purchased from Chemexpress Co., Ltd. (China) and suspended in 1% methylcellulose.

### Animals

Six-week-old female C57BL/6J mice were obtained from Japan SLC, Inc. (Japan). Animals were kept in an animal room facility at a temperature of 23 ± 2°C, humidity of 45 ± 10%, under a 12-hour light and dark cycle (light from 8:00 to 20:00). A high pressure of 20 ± 4 Pa was kept in the experimental room to prevent contamination. The animals were housed in TPX cages (CLEA Japan; maximum of 6 mice per cage) and fed with a sterilized normal diet (CE-2; CLEA Japan, Japan) provided ad libitum, being placed in a metal lid on the top of the cage. Sterilized Paper-Clean (Japan SLC) was used for bedding and was replaced once a week. Pure water was provided ad libitum from a water bottle equipped with a rubber stopper and a sipper tube.

### Induction of Bleomycin (BLM)-induced pulmonary fibrosis model

On Day 0, 48 mice were anesthetized with pentobarbital sodium (Kyoritsu Seiyaku, Japan) and intratracheally administered BLM (Lot # 761820, Nippon Kayaku, Japan) in saline at a dose of 3 mg/kg, in a volume of 50 µL per animal using a Microsprayer® (Penn-Century, USA). The mice were transferred to a clean cage (resting cage) and kept until recovery from anesthesia. The BLM administration took place on two separate days, with equal numbers of mice assigned to each day. Mice were randomized into 4 groups of 6 mice based on their body weight changes on the day before the start of treatment at Day 7. Control mice were intratracheally administered saline, instead of the BLM, and served as the Control group.

### Experimental Design

The design involved 60 female C57BL/6J mice, 12 mice that have received saline and 48 mice that have received BLM, as described above. The animals were divided into groups and treated according to the design of Table 8 below. Compound B (at doses of 7 or 14 mg/kg) or vehicle were administered subcutaneously (SC) during days 7-20 (Groups 2-4). Nintedanib was given per os at a daily dose of 100 mg/kg (Group 5). Animals were sacrificed at Day 21.

**Table 8. Groups and Treatments**

| Group | No. mice | Mice | Test substance | Dose (mg/kg) | Volume (mL/kg) | Regimen | Sacrifice |
|---|---|---|---|---|---|---|---|
| 1 | 12 | Normal | - | - | - | - | Day 21 |
| 2 | 12 | BLM | Vehicle | - | 7 | Subcutaneous, daily Day 7-20 | Day 21 |
| 3 | 12 | BLM | Compound B | 14 | 7 | Subcutaneous, daily Day 7-20 | Day 21 |
| 4 | 12 | BLM | Compound B | 7 | 3.5 | Subcutaneous, daily Day 7-20 | Day 21 |
| 5 | 12 | BLM | Nintedanib | 100 | 10 | Per os, daily Day 7-20 | Day 21 |

### Group Assessments

The viability, clinical signs and behavior were monitored every day. Body weight was recorded daily after the day of starting the BLM administration (Day 0). Animals were sacrificed by exsanguination through the abdominal aorta under pentobarbital sodium anesthesia and according to standard ethical procedures.

### Sample collection, assays and measurements

### Measurement of lung hydroxyproline content

To quantify lung hydroxyproline content, frozen whole left lung samples were processed by an acid hydrolysis method as follows. Lung samples were acid-hydrolyzed with 300 µL of 6N HCl at 121°C for 20 minutes, and neutralized with 300 µL of 4N NaOH containing 10 mg/mL activated carbon. AC buffer (2.2M acetic acid/0.48M citric acid, 300 µL) was added to the samples, followed by centrifugation to collect the supernatant. A standard curve of hydroxyproline was constructed with serial dilutions of trans-4-hydroxy-L-proline (Sigma-Aldrich Co. LLC., USA) starting at 16 µg/mL. The prepared samples and standards (each 400 µL) were mixed with 400 µL chloramine T solution (Wako Pure Chemical Industries, Ltd.) and incubated for 25 minutes at room temperature. The samples were then mixed with Ehrlich's solution (400 µL) and heated at 65°C for 20 minutes to develop the color. After samples were cooled on ice and centrifuged to remove precipitates, the optical density of each supernatant was measured at 560 nm. The concentrations of hydroxyproline were calculated from the hydroxyproline standard curve. Lung hydroxyproline levels were expressed as µg per left lung.

### Histopathological analysis

Right lung tissues prefixed in 10% neutral buffered formalin were embedded in paraffin and sectioned at 4 µm. For Masson's Trichrome staining, the sections were stained with Masson's Trichrome staining Kit (Sigma, USA) according to the manufacturer's instructions. The degree of pulmonary fibrosis was evaluated using the Ashcroft score (Ashcroft, T., et al., J Clin Pathol, 1988; 41:467-70) for the quantitative histological analysis.

### Sample collection

For frozen lung samples, the post caval lobe was collected, snap frozen in liquid nitrogen and stored at -80°C. Paraffin-embedded lung tissue blocks were stored at room temperature.

### Statistical tests

Statistical analyses were performed using Prism Software 6 (GraphPad Software, USA). For survival analysis, the Kaplan-Meier analysis with the Log-rank test was performed. For other data, statistical analyses were performed using Bonferroni Multiple Comparison Test. P values < 0.05 were considered statistically significant. A trend or tendency was assumed when a one-tailed t-test returned P values < 0.1. All results were expressed as mean ± SD.

### Body weight changes and general condition

Body weight was expressed as percentage of body weight change from baseline (Day 0). Mean body weight changes of the Vehicle group was significantly lower than that of the Control group from Day 6 to 11. Mean body weight changes of the Compound B 7 mg/kg group was significantly lower than that of the Vehicle group on Day 14. There were no significant differences in mean body weight changes at any day during the study period between the Vehicle group and the other treatment groups (data not shown).

Mean body weight changes at sacrifice of the Vehicle group tended to decrease compared with the Control group. There were no significant differences in mean body weight changes at sacrifice between the Vehicle group and the treatment groups (Table 1). During the treatment period, mice found dead before reaching Day 21 were as follows; three out of 12 mice were found dead in the Vehicle and Nintedanib groups. Four out of 12 mice were found dead in the Compound B groups. The mortality rate observed in this experiment in all groups is a standard model feature and the deaths observed are within the historical range for BLM model mice.

**Table 9. Body weight changes at sacrifice**

| Parameter | Control | Vehicle | Compound B 14 mg/kg | Compound B 7 mg/kg | Nintedanib 100 mg/kg |
|---|---|---|---|---|---|
| (mean ± SD) | (n=12) | (n=9) | (n=8) | (n=8) | (n=9) |
| Body weight changes (%) | 111 ± 5 | 106 ± 5 | 107 ± 6 | 107 ± 8 | 103 ± 5 |

### Survival analysis

There was no significant difference in survival rate between the Control group and the Vehicle group. There were no significant differences in survival rate between the Vehicle group and the treatment groups (data not shown).

### Lung hydroxyproline content (Table 10)

The Vehicle group showed a significant increase in lung hydroxyproline content compared with the Control group (P<0.05). Lung hydroxyproline contents in the Compound B, 7 and 14 mg/kg, and Nintedanib groups tended to decrease compared with the Vehicle group (P< 0.1).

**Table 10. Lung hydroxyproline content**

| Parameter | Control | Vehicle | Compound B 14 mg/kg | Compound B 7 mg/kg | Nintedanib 100 mg/kg |
|---|---|---|---|---|---|
| (mean ± SD) | (n=12) | (n=9) | (n=8) | (n=8) | (n=9) |
| Lung Hydroxyproline (µg/ml left lung) | 38.8 ± 2.9 | 59.6 ± 8.2 | 50.4 ± 10.2 | 48.4 ± 8.4 | 50.6 ± 14.5 |

### Histological analysis

### Masson's Trichrome staining and Ashcroft score

Table 11 shows Ashcroft scores determined as based on Masson's Trichrome stained tissues. In these assays, the Vehicle group showed a significant increase in Ashcroft score compared with the Control group (P<0.05). Ashcroft score in the Nintedanib group tended to decrease compared with the Vehicle group (P<0.1). There were no significant differences in Ashcroft score between the Vehicle group and the Compound B treatment groups although scores were reduced in both groups of Compound B treated animals.

**Table 11. Histopathological analysis**

| Parameter | Control | Vehicle | Compound B 14 mg/kg | Compound B 7 mg/kg | Nintedanib 100 mg/kg |
|---|---|---|---|---|---|
| (mean ± SD) | (n=12) | (n=9) | (n=8) | (n=8) | (n=9) |
| Ashcroft score | 0.1 ± 0.1 | 2.7 ± 1.2 | 2.0 ± 1.3 | 2.2 ± 1.2 | 1.6 ± 1.0 |

This example shows at least the following: as evidenced by lung hydroxyproline content and Ashcroft score, pulmonary fibrosis was established in the Vehicle group in the present study. Treatment with Compound B showed decreasing trends in lung hydroxyproline contents compared with the Vehicle group (P<0.1). A general reduction was seen also in Ashcroft scores. Treatment with Nintedanib showed decreasing trends in lung hydroxyproline contents and Ashcroft score compared with the Vehicle group (P<0.1). Taking into account that nintedanib is a clinically approved drug for the indication, these results suggest that Compound B has anti-fibrotic activity in the BLM model.

## Claims

1. A compound of formula (IV)
R-Y1-CH₂-CO-X (IV)
wherein R is a linear C₁₀₋₂₄ unsubstituted unsaturated alkylene group said group comprising at least 4 non-conjugated double bonds;
X is CF₃; and
Y1 is selected from O, S, SO or SO₂; or a salt thereof
for use in the treatment or prevention of a fibrotic disease, wherein the fibrotic disease is pulmonary fibrotic disorder or liver fibrosis.

2. A compound for use as claimed in claim 1 wherein said R group has 5 to 7 double bonds.

3. A compound for use as claimed in any preceding claim wherein in formula (IV) no double bond is conjugated with the carbonyl group.

4. A compound for use as claimed in any preceding claim wherein in said R group all double bonds are in the cis configuration or all double bonds are in the cis configuration except the double bond nearest the carbonyl.

5. A compound for use as claimed in any preceding claim wherein the R group comprises 17 to 19 carbon atoms.

6. A compound for use as claimed in any preceding claim wherein R is linear.

7. A compound for use as claimed in any preceding claim wherein said compound of formula (IV) has the formula: 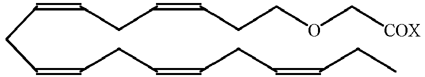 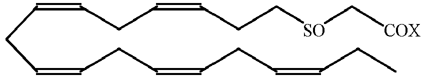 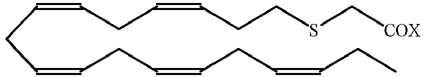 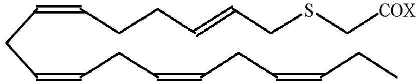 wherein X is CF₃.

8. A compound for use as claimed in any preceding claim where the compound of formula (IV) is Compound A or Compound B: 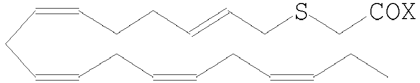 X= CF₃ = Compound A 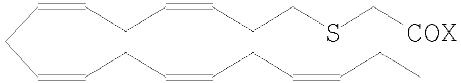 X= CF₃ = Compound B

9. A compound for use as claimed in any preceding claim wherein the fibrotic disease is due to injury or is idiopathic.

10. A compound for use as claimed in claim 9 wherein the injury is an ischemic event or due to exposure to radiation, a chemical, or an infectious agent.

11. A compound for use as claimed in any preceding claim wherein the compound is administered after a fibrotic lesion has developed in the subject.

12. A compound for use as claimed in any preceding claim wherein the compound is formulated with a pharmaceutically acceptable excipient.

13. A compound for use as claimed in any preceding claim wherein the compound is administered in combination with one or more adjunctive therapeutic agents.

14. A compound for use as claimed in any preceding claim wherein the pulmonary fibrotic disorder is one of idiopathic pulmonary fibrosis, interstitial lung disease, interstitial pulmonary fibrosis, chronic interstitial pneumonitis, Hamman-Rich Syndrome, usual interstitial pneumonitis (UIP), fibrosing alveolitis, pulmonary sarcoidosis, progressive massive fibrosis of lung, systemic sclerosis of lung or lung transplant associated fibrosis.

15. A compound of formula (IV) or a salt thereof as defined in claim 1 to 8 for use as a medicament for reducing one or more of hydroxyproline content or collagen Type 1 mRNA expression in the lung or liver of an animal.

## Patentansprüche

1. Verbindung der Formel (IV)
R-Y1-CH₂-CO-X (IV)
wobei R eine lineare unsubstituierte ungesättigte C₁₀₋₂₄-Alkylengruppe ist, wobei die Gruppe mindestens 4 nicht-konjugierte Doppelbindungen umfasst;
X CF₃ ist; und
Y1 ausgewählt ist aus O, S, SO oder SO₂; oder einem Salz davon zur Verwendung bei der Behandlung oder Vorbeugung einer fibrotischen Erkrankung, wobei die fibrotische Erkrankung eine fibrotische Lungenerkrankung oder Leberfibrose ist.

2. Verbindung zur Verwendung nach Anspruch 1, wobei die R-Gruppe 5 bis 7 Doppelbindungen aufweist.

3. Verbindung zur Verwendung nach einem vorstehenden Anspruch, wobei in Formel (IV) keine Doppelbindung mit der Carbonylgruppe konjugiert ist.

4. Verbindung zur Verwendung nach einem vorstehenden Anspruch, wobei in der R-Gruppe alle Doppelbindungen in der cis-Konfiguration vorliegen oder alle Doppelbindungen in der cis-Konfiguration vorliegen, mit Ausnahme der Doppelbindung, die dem Carbonyl am nächsten ist.

5. Verbindung zur Verwendung nach einem vorstehenden Anspruch, wobei die R-Gruppe 17 bis 19 Kohlenstoffatome umfasst.

6. Verbindung zur Verwendung nach einem vorstehenden Anspruch, wobei R linear ist.

7. Verbindung zur Verwendung nach einem vorstehenden Anspruch, wobei die Verbindung der Formel (IV) die folgende Formel aufweist: 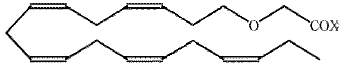 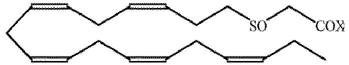 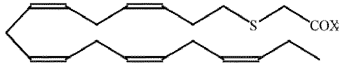 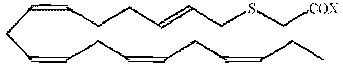 wobei X CF₃ ist.

8. Verbindung zur Verwendung nach einem vorstehenden Anspruch, wobei die Verbindung der Formel (IV) die Verbindung A oder Verbindung B ist: 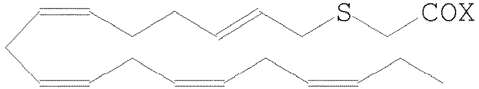 X= CF₃ = Verbindung A 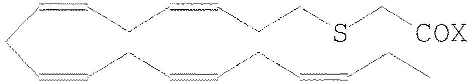 X= CF₃ = Verbindung B

9. Verbindung zur Verwendung nach einem vorstehenden Anspruch, wobei die fibrotische Erkrankung auf eine Verletzung zurückzuführen oder idiopathisch ist.

10. Verbindung zur Verwendung nach Anspruch 9, wobei die Verletzung ein ischämisches Ereignis ist oder auf die Exposition gegenüber Strahlung, einer Chemikalie oder einem infektiösen Agens zurückzuführen ist.

11. Verbindung zur Verwendung nach einem vorstehenden Anspruch, wobei die Verbindung verabreicht wird, nachdem sich bei dem Patienten eine fibrotische Läsion entwickelt hat.

12. Verbindung zur Verwendung nach einem vorstehenden Anspruch, wobei die Verbindung mit einem pharmazeutisch verträglichen Hilfsstoff formuliert ist.

13. Verbindung zur Verwendung nach einem vorstehenden Anspruch, wobei die Verbindung in Kombination mit einem oder mehreren adjunktiven therapeutischen Wirkstoffen verabreicht wird.

14. Verbindung zur Verwendung nach einem vorstehenden Anspruch, wobei die fibrotische Lungenerkrankung eine von idiopathischer Lungenfibrose, interstitielle Lungenerkrankung, interstitielle Lungenfibrose, chronische interstitielle Pneumonitis, Hamman-Rich-Syndrom, gewöhnliche interstitielle Pneumonitis (UIP), fibrosierende Alveolitis, pulmonale Sarkoidose, progressive massive Lungenfibrose, systemische Lungensklerose oder lungentransplantat-assoziierte Fibrose ist.

15. Verbindung der Formel (IV) oder ein Salz davon nach Anspruch 1 bis 8 zur Verwendung als Medikament zur Verringerung eines oder mehrerer von Hydroxyprolin-Gehalt oder Kollagen-Typ-1-mRNA-Expression in der Lunge oder Leber eines Tieres.

## Revendications

1. Composé de formule (IV)
R-Y1-CH₂-CO-X (IV)
dans lequel R est un groupe alkylène insaturé non substitué linéaire en C₁₀₋₂₄, ledit groupe comprenant au moins 4 doubles liaisons non conjuguées ;
X est CF₃ ; et
Y1 est choisi parmi O, S, SO ou SO₂; ou un sel de ceux-ci
à utiliser dans le traitement ou la prévention d'une maladie fibreuse, dans lequel la maladie fibreuse est un trouble fibreux pulmonaire ou une fibrose hépatique.

2. Composé pour utilisation selon la revendication 1 dans lequel ledit groupe R présente 5 à 7 doubles liaisons.

3. Composé pour utilisation selon une quelconque revendication précédente dans lequel, dans la formule (IV), aucune double liaison n'est conjuguée au groupe carbonyle.

4. Composé pour utilisation selon une quelconque revendication précédente dans lequel, dans ledit groupe R, toutes les doubles liaisons sont dans la configuration cis ou toutes les doubles liaisons sont dans la configuration cis, à l'exception de la double liaison la plus proche du carbonyle.

5. Composé pour utilisation selon une quelconque revendication précédente dans lequel le groupe R comprend 17 à 19 atomes de carbone.

6. Composé pour utilisation selon une quelconque revendication précédente dans lequel R est linéaire.

7. Composé pour utilisation selon une quelconque revendication précédente dans lequel ledit composé de formule (IV) présente la formule : 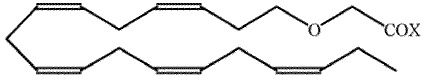 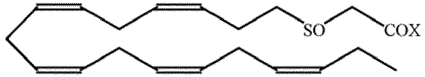 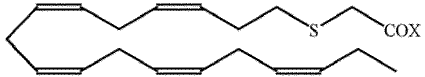 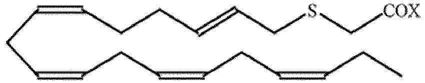 dans lequel X est CF₃.

8. Composé pour utilisation selon une quelconque revendication précédente dans lequel le composé de formule (IV) est un Composé A ou Composé B : 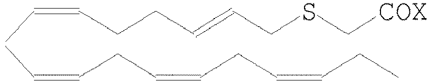 X= CF₃ = Composé A 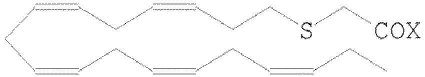 X = CF₃ = Composé B.

9. Composé pour utilisation selon une quelconque revendication précédente dans lequel la maladie fibreuse est due à une blessure ou est idiopathique.

10. Composé pour utilisation selon la revendication 9 dans lequel la blessure est un événement ischémique ou est due à une exposition à un rayonnement, à un produit chimique, ou à un agent infectieux.

11. Composé pour utilisation selon une quelconque revendication précédente dans lequel le composé est administré après développement d'une lésion fibreuse chez le sujet.

12. Composé pour utilisation selon une quelconque revendication précédente dans lequel le composé est formulé avec un excipient pharmaceutiquement acceptable.

13. Composé pour utilisation selon une quelconque revendication précédente dans lequel le composé est administré en combinaison avec un ou plusieurs agents thérapeutiques adjuvants.

14. Composé pour utilisation selon une quelconque revendication précédente dans lequel le trouble fibreux pulmonaire est l'un parmi une fibrose pulmonaire idiopathique, une maladie pulmonaire interstitielle, une fibrose pulmonaire interstitielle, une pneumopathie interstitielle chronique, un syndrome de Hamman-Rich, une pneumopathie interstitielle commune (UIP), une alvéolite fibrosante, une sarcoïdose pulmonaire, une fibrose massive progressive du poumon, une sclérose systémique du poumon ou fibrose associée à une greffe de poumon.

15. Composé de formule (IV) ou sel de celui-ci tel que défini dans les revendications 1 à 8 pour une utilisation en tant que médicament pour réduire une ou plusieurs parmi une teneur en hydroxyproline ou une expression d'ARNm de collagène de Type 1 dans le poumon ou le foie d'un animal.


### SEQUENCE LISTING

<110> Avexxin AS
<120> COMPOSITIONS AND METHODS FOR TREATMENT OF A FIBROTIC DISEASE
<130> 3.133525/02
<150> GB 1709671.0
   <151> 2017-06-16
<150> GB 1806661.3
   <151> 2018-04-24
<160> 28
<170> PatentIn version 3.5
<210> 1
   <211> 20
   <212> DNA
   <213> Artificial Sequence
<220>
   <223> Primer sequence
<400> 1
   tgtgtccgtc gtggatctga 20
<210> 2
   <211> 21
   <212> DNA
   <213> Artificial Sequence
<220>
   <223> Primer sequence
<400> 2
   ttgctgttga agtcgcagga g 21
<210> 3
   <211> 24
   <212> DNA
   <213> Artificial Sequence
<220>
   <223> Primer sequence
<400> 3
   ccaacaagca tgtctggtta ggag 24
<210> 4
   <211> 23
   <212> DNA
   <213> Artificial Sequence
<220>
   <223> Primer sequence
<400> 4
   gcaatgctgt tcttgcagtg gta 23
<210> 5
   <211> 20
   <212> DNA
   <213> Artificial Sequence
<220>
   <223> Primer sequence
<400> 5
   gccatcagga acctcgagaa 20
<210> 6
   <211> 21
   <212> DNA
   <213> Artificial Sequence
<220>
   <223> Primer sequence
<400> 6
   ggagcatcat caccagcaaa g 21
<210> 7
   <211> 21
   <212> DNA
   <213> Artificial Sequence
<220>
   <223> Primer sequence
<400> 7
   ctcatactga taggagagac g 21
<210> 8
   <211> 20
   <212> DNA
   <213> Artificial Sequence
<220>
   <223> Primer sequence
<400> 8
   tcgaacttga gtttgaagtg 20
<210> 9
   <211> 20
   <212> DNA
   <213> Artificial Sequence
<220>
   <223> Primer sequence
<400> 9
   agtggaagag cgattactac 20
<210> 10
   <211> 20
   <212> DNA
   <213> Artificial Sequence
<220>
   <223> Primer sequence
<400> 10
   attgatggtc tctcctaacc 20
<210> 11
   <211> 20
   <212> DNA
   <213> Artificial Sequence
<220>
   <223> Primer sequence
<400> 11
   agtggccata atggggaacg 20
<210> 12
   <211> 20
   <212> DNA
   <213> Artificial Sequence
<220>
   <223> Primer sequence
<400> 12
   cagggtttcc atcccttccg 20
<210> 13
   <211> 22
   <212> DNA
   <213> Artificial Sequence
<220>
   <223> Primer sequence
<400> 13
   ggacactatg cgggtcactt gg 22
<210> **14**
   <211> 22
   <212> DNA
   <213> Artificial Sequence
<220>
   <223> Primer sequence
<400> 14
   tgctgttcgt acacgctgga ga 22
<210> 15
   <211> 20
   <212> DNA
   <213> Artificial Sequence
<220>
   <223> Primer sequence
<400> 15
   cgattccgtg ggtggtggtg 20
<210> 16
   <211> 24
   <212> DNA
   <213> Artificial Sequence
<220>
   <223> Primer sequence
<400> 16
   catgccagag tctcgttcgt tatc 24
<210> 17
   <211> 19
   <212> DNA
   <213> Artificial Sequence
<220>
   <223> Primer sequence
<400> 17
   agatcaagat cattgcccc 19
<210> 18
   <211> 20
   <212> DNA
   <213> Artificial Sequence
<220>
   <223> Primer sequence
<400> 18
   ttcatcgtat tcctgtttgc 20
<210> 19
   <211> 21
   <212> DNA
   <213> Artificial Sequence
<220>
   <223> Primer sequence
<400> 19
   aagcaggcta atactgatag g 21
<210> 20
   <211> 20
   <212> DNA
   <213> Artificial Sequence
<220>
   <223> Primer sequence
<400> 20
   tgttgaaaag tagttctggg 20
<210> 21
   <211> 20
   <212> DNA
   <213> Artificial Sequence
<220>
   <223> Primer sequence
<400> 21
   aaagggagat caagggatag 20
<210> 22
   <211> 20
   <212> DNA
   <213> Artificial Sequence
<220>
   <223> Primer sequence
<400> 22
   tcaccttttt ctccaggtag 20
<210> 23
   <211> 20
   <212> DNA
   <213> Artificial Sequence
<220>
   <223> Primer sequence
<400> 23
   attcacctac acagttctgg 20
<210> 24
   <211> 20
   <212> DNA
   <213> Artificial Sequence
<220>
   <223> Primer sequence
<400> 24
   tgcgtgttcg atattcaaag 20
<210> 25
   <211> 20
   <212> DNA
   <213> Artificial Sequence
<220>
   <223> Primer sequence
<400> 25
   ttaagaaggg caaaaagtgc 20
<210> 26
   <211> 22
   <212> DNA
   <213> Artificial Sequence
<220>
   <223> Primer sequence
<400> 26
   catactccac agaatttagc tc 22
<210> 27
   <211> 20
   <212> DNA
   <213> Artificial Sequence
<220>
   <223> Primer sequence
<400> 27
   cagaaggatg taaaggatgg 20
<210> 28
   <211> 20
   <212> DNA
   <213> Artificial Sequence
<220>
   <223> Primer sequence
<400> 28
   tatttcttct tggacacacc 20